# FASCICULE DE BREVET EUROPEEN

(11) **EP 1 899 485 B1**
(45) Date de publication et mention de la délivrance du brevet: **14.04.2010**
(21) Numéro de dépôt: 06778613.7
(22) Date de dépôt: 19.06.2006
(51) Int. Cl.: C12Q 1/68

(54) **PROCEDE D'IMMOBILISATION DE L'ADN SUPERENROULE ET UTILISATION POUR ANALYSER LA REPARATION DE L'ADN**
VERFAHREN ZUR FIXIERUNG EINER SUPERHELIKALEN DNA UND VERWENDUNG ZUR ANALYSE DER DNA-REPARATUR
METHOD FOR FIXING A SUPERCOILED DNA AND THE USE FOR ANALYSING THE DNA REPAIR

(30) Priorité: 20.06.2005 FR 0506213
(43) Date de publication de la demande: 19.03.2008
(73) Titulaire: COMMISSARIAT A L'ENERGIE ATOMIQUE, 75015 Paris (FR)
(72) Inventeur: MILLAU, Jean-François, 07110 CHAASIERS (FR); SAUVAIGO, Sylvie, 38000 Grenoble (FR)
(74) Mandataire: Leblois-Préhaud, Hélène Marthe Georgette
(86) Numéro de dépôt international: PCT/FR2006/001378
(87) Numéro de publication internationale: WO 2006/136686

(56) Documents cités:
- WO-A-99/49080
- FR-A- 2 849 058
- US-A1- 2002 022 228
- US-B1- 6 309 838
- KAWAMURA M ET AL: "A NEW APPROACH TO THE DETECTION OF DNA DAMAGE" LEUKEMIA RESEARCH, vol. 13, no. 5, 1989, pages 391-398, XP002359927 ISSN: 0145-2126

## Description

L'invention concerne un procédé d'immobilisation de l'ADN superenroulé et l'utilisation de l'ADN superenroulé immobilisé sur un support, comme substrat pour analyser les réactions enzymatiques de réparation de l'ADN.

Les biopuces sont devenues des outils d'analyse indispensables pour la recherche biomédicale et le diagnostic clinique. Ces outils puissants permettent d'analyser simultanément plusieurs dizaines à plusieurs centaines de milliers d'échantillons, à l'aide de sondes à ADN (oligonucléotides, ADNc, produits d'amplification PCR) ou à protéine (anticorps, peptides), immobilisées sur un support.

Les biopuces sont préparées à l'aide d'un support (verre, polypropylène, polystyrène, silicone, métal, nitrocellulose ou nylon), éventuellement modifié par un film poreux [verre recouvert de nylon (Atlas arrays™; CLONTECH) ; silicone recouverte d'un hydrogel (Nanochip™; NANOGEN), verre recouvert d'un gel de polymère d'acrylamide HydroGel™ (PERKIN-ELMER) ou de méthacrylate (Demande US 2005/0042363)].

Deux types de procédés sont utilisés pour immobiliser la sonde à ADN ou à protéine sur le support :

### * dépôt des sondes préalablement synthétisées et immobilisation par couplage covalent ou non-covalent

La sonde (oligonucléotide (10 à 25 bases), peptide, ADNc ou fragment d'amplification PCR (au maximum 500 pb à 0,2 kb), protéine (anticorps)) est généralement déposée sur le support à l'aide d'un distributeur automatique (Lemmo et al., Current Opinion in Biotechnology, 1998, 9:615-617). La sonde qui est chargée peut également être déposée à la surface d'un support à base de microélectrodes recouvertes d'une couche perméable (gel d'agarose) et transportée à l'intérieur du gel, par électrophorèse (Heller et al., Electrophoresis, 2000, 21:157-164).

En outre, dans le cas particulier de la technique d'hybridation de colonies sur filtre (ou *colony-hybridization;* Hanahan and Meselson, Gene, 1983, 10:63-67), des colonies de bactéries déposées sur un support sont transférées sur un filtre de nylon ou de nitrocellulose puis lysées de façon à libérer l'ADN (plasmide ou cosmide) sur le filtre.

La sonde est ensuite immobilisée sur le support par l'intermédiaire de liaisons covalentes ou non covalentes (hydrophobes, électrostatiques) ou d'une combinaison des deux.

Les liaisons covalentes sont généralement formées entre les groupements fonctionnels réactifs d'un support activé et ceux d'une sonde modifiée à l'une de ses extrémités (groupement phosphate ou acide carboxylique pour la sonde et amine pour le support, et réciproquement ; groupement amine pour la sonde et isothiocyanate, aldéhyde ou époxy, pour le support ; Zammateo et al., Analytical Biochemistry, 2000, 208: 143-150).

Lorsque le support est recouvert d'un film de polyacrylamide, il peut être activé par substitution de groupements hydrazide par traitement à l'hydrazine. Les sondes modifiées en 3' par un groupement 3-méthyl-uridine sont oxydées en présence de periodate pour former une aldéhyde réactive, puis elles sont déposées sur le support. La réaction des groupements aldéhyde de la sonde avec les groupements hydrazine du gel forme des liaisons covalentes qui immobilisent l'ADN dans le gel d'acrylamide (Khrapko et al; DNA sequence, 1991, 6:375-388; Yershov et al., P.N.A.S., 1996, 93:4913-4918). IL est également possible d'immobiliser des fragments d'ADN aminés, d'une longueur inférieure à 500 nucléotides, lors de la polymérisation du gel (Rubina et al., Anal. Biochem., 2004, 325:92-106).

Les supports recouverts d'hydrogel permettent notamment d'augmenter la capacité d'immobilisation du support et d'améliorer l'environnement de la sonde, la sensibilité et le seuil de détection.

En outre, l'irradiation aux UV ou des agents couplants homo ou bi-fonctionnels peuvent également former des liaisons covalentes entre les groupements amine d'un support recouvert de poly-L-lysine et, soit les résidus de thymidine de l'ADN (UV), soit les groupements amine d'une sonde modifiée (glutaraldéhyde).

Les liaisons non-covalentes comprennent principalement les interactions électrostatiques entre l'ADN et un support recouvert de poly-L-lysine.

### *synthèse et immobilisation des sondes in situ

Cette méthode utilisable uniquement pour la préparation de puces à oligonucléotides (Southern et al., Genomics, 1992, 4, 1008-1017) ou à peptides, permet de réaliser simultanément le couplage covalent et la synthèse des oligonucléotides sur une zone qui est délimitée, notamment à l'aide d'un masque (mécanique, photolithographique).

Ces procédés permettent d'obtenir des biopuces adaptées à de nombreuses applications analyse génétique (séquençage, cartographie, polymorphisme, mutations, nombre de copies de gènes, profil d'expression) et analyse d'interactions protéine/ligand (immunoessais ; interactions entre des protéines et des molécules d'ADN; d'ARN ou des petites molécules).

Toutefois, certaines analyses comme celle de l'activité de réparation de l'ADN, mettent en oeuvre une sonde à ADN particulière, à savoir un ADN double-brin circulaire superenroulé (plasmide superenroulé), dont la structure doit être impérativement préservée au cours de l'immobilisation de l'ADN sur le support, et conservée une fois que l'ADN a été immobilisé sur le support. En effet, le substrat utilisé pour analyser la réparation de l'ADN est un plasmide superenroulé présentant des lésions des bases puriques ou pyrimidiques (lésions oxydatives, photoproduits, adduits chimiques), des sucres, de la structure de la double-hélice (pontage inter ou intrabrins, agents intercalés), et/ou des cassures, induites par traitement du plasmide par différents agents génotoxiques, puis purification du plasmide superenroulé sur un gradient de saccharose ou de césium. La purification du plasmide superenroulé permet d'éliminer les plasmides comprenant des cassures (structure relachée). En effet, contrairement aux autres types de lésions précitées qui préservent la structure superenroulée de l'ADN, les cassures détruisent la structure superenroulée du plasmide et forment une structure relachée. Le plasmide superenroulé, non-traité (plasmide sans lésion) sert de contrôle. L'échantillon biologique à analyser est incubé en présence du plasmide superenroulé comprenant des lésions (plasmide lésé) et d'un nucléoside triphosphate marqué. L'activité de réparation de l'ADN est ensuite quantifiée par mesure de la quantité de marqueur incorporée dans le plasmide lésé, par comparaison avec le plasmide contrôle.

Les lésions non dirigées de l'ADN et notamment les cassures (simple- ou double-brin) apparues lors de l'immobilisation du plasmide sur un support et ultérieurement lors de la conservation du plasmide immobilisé sur un support, sont réparées par les systèmes de réparation contenus dans les échantillons à analyser. Ainsi la présence de cassures, en plus des bases modifiées, entraîne l'incorporation de marqueurs parasites qui sont quantifiés et masquent la réponse spécifique de réparation des bases. D'autre part, les cassures induisent un relâchement du plasmide et permettent ainsi un accès plus facile aux nucléases du milieu biologique, entraînant une augmentation de l'activité de réparation du plasmide. Enfin, les structures relâchées favorisent le démarrage de réaction de polymérisation non désirées. La réparation des lésions non dirigées de l'ADN et notamment des cassures peut ainsi masquer ou interférer avec la réparation de lésions spécifiques. Le signal spécifique des lésions étudiées risque ainsi d'être perdu dans le bruit de fond engendré par les signaux non spécifiques, rendant l'analyse de la réparation de l'ADN difficile, voire impossible.

La plupart des procédés de préparation de biopuces ne permettent pas d'immobiliser de l'ADN de grande taille présentant une structure particulière comme les plasmides (ADN circulaire superenroulé), en préservant leur intégrité.

En effet, l'immobilisation de l'ADN superenroulé par couplage non-covalent sur des lames recouvertes de poly-L-lysine telle que décrite dans la Demande de Brevet FR 02 16435, ne permet pas de préserver la structure de l'ADN de façon stable et les supports comprenant le plasmide immobilisé doivent être utilisés immédiatement après l'immobilisation de l'ADN, ce qui limite leur utilisation industrielle. Les interactions entre l'ADN dont les groupements phosphates internucléosidiques sont chargés négativement, et les groupements cationiques de la poly-L-lysine, entraînent plus ou moins rapidement une instabilité de la structure superenroulée du plasmide conduisant à l'apparition de cassures dans l'ADN et à l'amorçage de réactions de réparation non désirées. En conséquence, on note une augmentation du taux de réparation du plasmide non modifié servant de contrôle, quelques heures après le dépôt sur les lames poly-Llysine, par rapport à une réaction effectuée immédiatement après le dépôt. Ce taux de réparation détectée sur le plasmide contrôle augmente proportionnellement au temps de conservation des lames. Le même phénomène se produit sur les plasmides comportant des lésions de l'ADN et masque la réponse spécifique attendue. Aussi, les lames doivent être utilisées très rapidement après le dépôt des plasmides, ce qui présente un inconvénient majeur pour une utilisation industrielle.

La conservation des lames poly-L-lysine sur lesquelles sont immobilisées des plasmides est par ailleurs très dépendante de l'environnement hygrométrique lors du dépôt des plasmides et du stockage des lames. Or le contrôle de ce paramètre est délicat à effectuer de façon précise, ce qui conduit à des durées de conservation aléatoires des lames. Le caractère aléatoire de la conservation limite davantage l'utilisation de cette technique. De plus, même si le dépôt du plasmide peut éventuellement se faire en atmosphère humide, cette alternative n'est pas satisfaisante puisque l'accrochage du plasmide sur lame poly-L-lysine est moins bon et les signaux de réparation obtenus sont faibles.

Un autre désavantage de cette méthode est lié au volume des dépôts des solutions de plasmide sur la lame. Celui-ci est de l'ordre de quelques centaines de picolitres ; or, la vitesse d'évaporation de l'eau contenue dans les dépôts étant dépendante de facteurs tels que le taux d'humidité ambiante, la température ambiante et les adjuvants éventuellement présents dans la solution de dépôt, il y a toujours un risque qu'une évaporation rapide de l'eau entraîne une concentration de l'échantillon à la circonférence de la goutte et donc un manque d'homogénéité du dépôt. Le manque d'homogénéité du dépôt a pour conséquence des difficultés lors de la quantification du signal de réparation au niveau du dépôt, alors qu'une quantification précise est nécessaire pour la détermination des capacités de réparation d'un milieu cellulaire donné et pour comparer différents milieux entre eux. Rickman et al. (Nucleic Acids Res., 2003, 31, e109) ont toutefois montré que certains adjuvants ralentissent l'évaporation. Cependant l'utilisation de tels composés entraîne une hétérogénéité de morphologie ou une augmentation de la taille des dépôts. Certains composés semblent donner des résultats intéressants en terme de qualité de dépôt mais ne sont pas adaptés aux études de réparation car ils sont susceptibles d'entraîner une dénaturation du plasmide comme c'est le cas de la formamide (Rickman et al, 2003, précité).

Le couplage covalent de l'ADN sur le support n'est pas envisageable dans la mesure où il génère des tensions conduisant à l'apparition de cassures sur le plasmide. En outre, il implique des étapes de traitement chimique et/ou physique (chauffage, irradiation par les UV) susceptibles d'introduire des lésions dans l'ADN. De plus, les méthodes de synthèse *in situ* sont utilisables uniquement pour la préparation de puces à oligonucléotides.

En conséquence, il existe un réel besoin de disposer de procédés alternatifs pour immobiliser l'ADN superenroulé sur un support, qui répondent mieux aux besoins dans le domaine en ce qu'ils respectent l'intégrité de la structure superenroulée de l'ADN et qu'ils permettent de conserver cette structure en vue d'une utilisation ultérieure de l'ADN, notamment comme substrat dans les réactions de réparation enzymatique de l'ADN.

Les Inventeurs ont montré que l'ADN superenroulé pouvait être immobilisé de façon stable dans un film de polymère poreux, par diffusion passive de l'ADN non activé par des groupements fonctionnels réactifs (non fonctionnalisé), dans un film de polymère, également non activé. Dans ces conditions, l'ADN immobilisé conserve sa structure superenroulée pendant plusieurs mois, même lorsqu'il comprend des lésions introduites par l'exposition à des agents génotoxiques. L'ADN est avantageusement immobilisé sur un support miniaturisé recouvert d'un film de polymère poreux, permettant la préparation en grande quantité d'échantillons d'ADN immobilisé et leur stockage en vue d'une utilisation ultérieure notamment comme substrat dans des réactions de réparation de l'ADN.

La présente invention a en conséquence pour objet, un procédé d'immobilisation d'un ADN superenroulé, caractérisé en ce qu'il comprend les étapes suivantes:
- le dépôt d'un échantillon d'ADN superenroulé à la surface d'un film de polymère poreux, et
- l'immobilisation de l'ADN superenroulé par diffusion passive dans ledit film de polymère poreux.

### Définitions

- ADN superenroulé : une molécule d'ADN double-brin circulaire superenroulée, notamment un plasmide.
- film de polymère poreux : une couche mince d'une matrice tridimensionnelle comprenant des chaînes d'un ou plusieurs polymères et un solvant, laquelle matrice incluant des interstices (pores) pouvant contenir des macromolécules comme l'ADN. L'épaisseur du film de polymère est suffisamment faible pour limiter le bruit de fond. De préférence, l'épaisseur du film est inférieure à 0,1 mm.
- immobilisation : la pénétration de l'ADN par diffusion passive au sein de la matrice tridimensionnelle constituée de chaînes d'un ou plusieurs polymère(s) et d'un solvant.

Dans ces conditions l'ADN superenroulé, dilué dans un tampon classique connu de l'homme du métier, notamment du PBS, pH 7,4, pénètre dans le film de polymère poreux en l'absence de courant électrique (pas d'étape d'électrophorèse) et est immobilisé dans les interstices de la matrice de polymère, en l'absence de tout traitement physique ou chimique ultérieur du film de polymère ou de l'ADN. L'ADN superenroulé ainsi immobilisé se maintient de façon stable dans le film de polymère, pendant plusieurs mois.

Parmi les polymères poreux préférés, on peut citer les polymères aqueux extensibles (hydrogel ou gel aqueux : polysaccharide et particulièrement l'agarose) et les polymères dans lesquels des liaisons chimiques covalentes sont établies entre les chaînes de polymères, de façon à former un maillage (polyacrylamide).

La quantité d'ADN immobilisé dans le polymère (capacité d'immobilisation) dépend de la nature du polymère et de sa concentration et peut être augmentée en ajoutant un adsorbant de l'ADN dans l'hydrogel.

Selon un premier mode de mise en oeuvre avantageux dudit procédé, le film de polymère est un hydrogel de polyacrylamide. De préférence, il comprend 5 à 15 % d'un mélange acrylamide:méthylène bisacrylamide 50:1 à 5 :1, de manière préférée 5 à 15 % d'un mélange 19: 1.

Selon un second mode de mise en oeuvre avantageux dudit procédé, le film de polymère comprend 0,1 % à 5 % d'un adsorbant de l'ADN tel que la gélatine, le collagène ou un polysaccharide comme l'agarose, le dextran ou un polymère de glucose ou de saccharose, ou bien un mélange desdits adsorbants. De préférence, ledit adsorbant est de l'agarose, de manière préférée un agarose à bas point de fusion *(low-melting agarose*) dont la température de fusion est avantageusement inférieure à 40°C.

Selon une disposition avantageuse des modes de mise en oeuvre précédents, le film de polymère est un hydrogel de polyacrylamide comprenant 10 % d'un mélange d'acrylamide:méthytène bisacrylamide 19:1 et 0,8 % d'agarose à bas point de fusion.

Selon un troisième mode de mise en oeuvre avantageux dudit procédé, ledit ADN superenroulé est un plasmide.

Selon un quatrième mode de mise en oeuvre avantageux dudit procédé, ledit ADN superenroulé comprend des lésions induites par un agent génotoxique. De préférence, lesdites lésions ont été induites par traitement d'un plasmide isolé ou de cellules comprenant ledit plasmide, par un agent physique ou chimique génotoxique. Parmi ces lésions, on peut citer notamment les lésions des bases puriques ou pyrimidiques (lésions oxydatives, photoproduits, adduits chimiques), des sucres, de la structure de la double-hélice (pontage inter ou intrabrins, agents intercalés) et les cassures.

Selon un cinquième mode de mise en oeuvre avantageux dudit procédé, le film de polymère poreux est déposé sur un support approprié connu de l'Homme du métier, tel qu'un support en verre, en métal, en silicone ou en plastique. De préférence, il s'agit d'un support miniaturisé du type micropuce. De manière préférée, il s'agit d'une lame de verre.

La présente invention a également pour objet un support recouvert d'un film de polymère poreux comprenant un ADN superenroulé immobilisé, susceptible d'être obtenu par le procédé d'immobilisation tel que défini ci-dessus.

Avantageusement, il s'agit d'une lame de verre recouverte d'un hydrogel de polyacrylamide comprenant un ADN superenroulé lésé.

La polymérisation et le dépôt du film de polymère à la surface d'un support sont réalisés selon les techniques classiques connues de l'Homme du métier. Toutes les techniques connues peuvent être utilisées pour la mise en oeuvre de l'invention. Un polymère poreux tel qu'un hydrogel de polyacrylamide est préparé à partir d'une solution aqueuse contenant de l'acrylamide et des moyens de polymérisation. Par exemple : (i) 5 à 15 % d'un mélange acrylamide:méthylène bisacrylamide (50:1 à 5:1, de préférence 19:1), (ii) 0 à 5 % d'adsorbant(s) de l'ADN tels que définis ci-dessus, de préférence un polysaccharide et particulièrement de l'agarose à bas point de fusion, et (iii) une source de radicaux, par exemple 0,001 à 0,1 % de persulfate d'ammonium en conjonction avec 0,001 à 0,5 % de TEMED ((N,N,N',N'-tetraméthyléthylène diamine).

Des compositions de polymères prêtes à l'emploi peuvent également être utilisées, notamment des hydrogels tels que HydroGeI^{™} (PERKIN-ELMER ; www.perkinelmer.com/proteomics).

Le support est par exemple une lame de verre préalablement enduite d'aminosilane. La solution aqueuse d'hydrogel est ensuite déposée et étalée sur le support, à l'aide d'un moyen adapté tel qu'une lamelle de verre, de façon à former une fine couche homogène. L'hydrogel est polymérisé, éventuellement par chauffage ou exposition aux ultraviolets, en fonction du moyen de polymérisation employé. Le support recouvert d'un film de polymère ainsi obtenu peut éventuellement être séché, de préférence par chauffage.

L'ADN, notamment un plasmide, est préparé selon les techniques classiques de biologie moléculaire, en utilisant les protocoles standards tels que décrits dans Current Protocols in Molecular Biology (Frederick M. AUSUBEL, 2000, Wiley and son Inc, Library of Congress, USA*).*

Le traitement de l'ADN par des agents génotoxiques est effectué selon les techniques classiques connues de l'Homme du métier, en suivant les protocoles standards tels que décrits dans la Demande de Brevet FR 0216435.

La fraction d'ADN superenroulée est isolée par centrifugation sur gradient de saccharose et/ou gradient de chlorure de césium, selon les protocoles standards tels que décrits dans la Demande de Brevet FR 0216435.

L'ADN superenroulé est dilué à une concentration comprise de préférence entre 5 et 100 µg/ml, et avantageusement de 20 à 40 µg/ml, dans un tampon standard tel que du PBS pH 7,4.

L'ADN superenroulé est ensuite déposé sur les zones du support recouvertes d'hydrogel, par exemple à l'aide d'un robot tel qu'un robot piézo-électrique.

La présente invention a également pour objet l'utilisation d'une composition aqueuse comprenant 10 % d'un mélange d'acrylamide:méthylène bisacrylamide 19:1 et 0,8 % d'agarose à bas point de fusion pour immobiliser de l'ADN superenroulé.

La présente invention a également pour objet l'utilisation d'un ADN superenroulé immobilisé dans un film de polymère poreux tel que défini ci-dessus, comme substrat pour analyser la réparation de l'ADN dans un milieu biologique.

La réparation de l'ADN peut notamment être analysée selon le procédé d'évaluation quantitative des capacités globales et spécifique de réparation de l'ADN décrit dans la Demande FR 0216435.

L'analyse de réparation de l'ADN dans un milieu biologique et notamment l'évaluation quantitative des capacités globales et spécifiques de réparation de l'ADN dans un milieu biologique, permet notamment d'établir le profil de réparation d'un milieu donné, de diagnostiquer une maladie liée à la réparation de l'ADN, d'évaluer l'influence d'un traitement physique ou chimique sur les capacités de réparation d'un milieu biologique donné et de cribler des substances capables de moduler le système de réparation d'un milieu biologique.

L'utilisation d'hydrogel à la place de la poly-L-lysine, pour immobiliser l'ADN superenroulé (ADN comprenant des lésions (plasmide superenroulé lésé) et ADN contrôle (plasmide superenroulé)), présente les avantages suivants, notamment pour mesurer l'activité de réparation de l'ADN dans un milieu biologique :

### - stabilité du plasmide immobilisé dans l'hydrogel :

Le plasmide immobilisé sur un support recouvert d'hydrogel, par exemple un polymère d'acrylamide, conserve sa structure superenroulée malgré les lésions de l'ADN, et ce pendant plusieurs semaines, dans un environnement non contrôlé du point de vue de l'hygrométrie. Ainsi de grandes quantités de lames comprenant le plasmide immobilisé peuvent être préparées simultanément et stockées aisément jusqu'à leur utilisation plusieurs semaines plus tard.

### - sensibilité de détection de la réparation de l'ADN

La gamme de valeurs du signal de réparation est plus large, ce qui permet une analyse plus précise des échantillons dont l'activité de réparation est analysée et notamment une meilleure discrimination des différences entre les échantillons analysés.

Outre les dispositions qui précèdent, l'invention comprend encore d'autres dispositions qui ressortiront de la description qui va suivre, qui se réfère à des exemples d'utilisation d'ADN superenroulé lésé, immobilisé dans un hydrogel, comme substrat de réaction de réparation enzymatique de l'ADN, ainsi qu'aux dessins annexés dans lesquels:

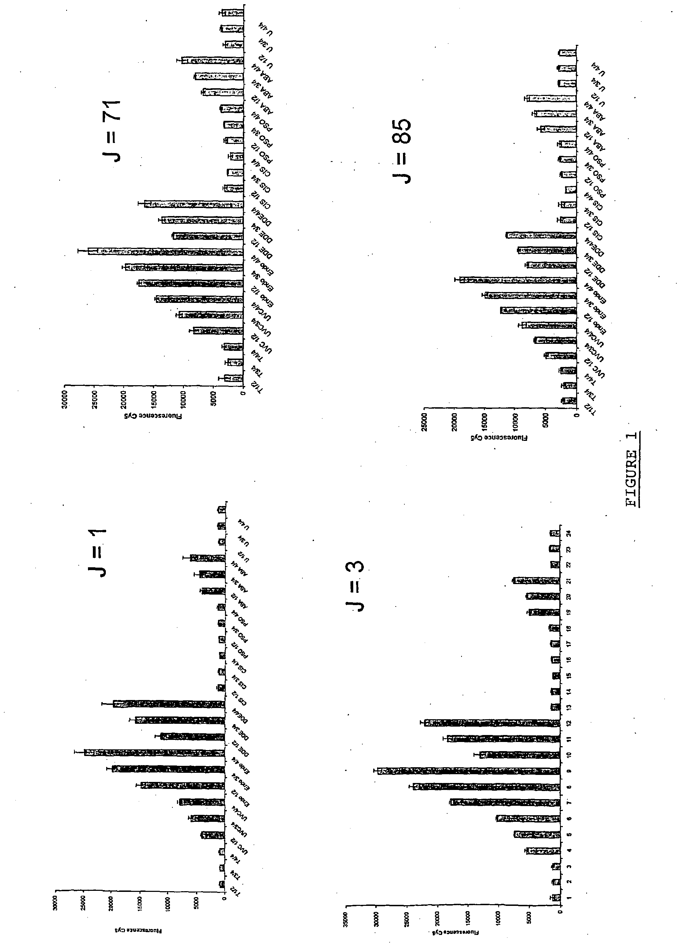

- la figure 1 illustre la stabilité à + 4°C, pendant une période de 85 jours, d'un ADN superenroulé lésé, immobilisé sur des lames de verre recouvertes d'un hydrogel de polyacrylamide. De l'ADN plasmidique superenroulé présentant des lésions induites par différents agents physiques ou chimiques a été préparé comme décrit à l'exemple 1, puis dilué à l'aide de plasmide contrôle (non-traité) pour conserver la concentration totale de plasmide dans le tampon PBS à 40 µg/ml : uniquement du plasmide lésé (**4/4**), 30 µg/ml de plasmide lésé et 10 µg/ml de plasmide contrôle (**3/4**) ; 20 µg/ml de plasmide lésé et 20 µg/ml de plasmide contrôle (**1/2**). De l'ADN plasmidique non-traité a été utilisé comme contrôle. Les échantillons d'ADN ont été déposés sur des lames de verre recouvertes d'un hydrogel de polyacrylamide puis les lames ont été conservées à +4°C pendant 1, 3, 71 et 85 jours (J₁, J₃, J₇₁ et J₈₅) avant d'évaluer la réparation de l'ADN, par mesure de l'émission de fluorescence. Les valeurs de fluorescence sont exprimées en unité arbitraire (u.a.). **T :** contrôle. **UVC :** irradiation aux UVC (0,3 J/cm²). **endo :** traitement à l'endoperoxyde de naphtalène. **DDE :** traitement au trans, trans-2,4-décadiénal. **CIS :** traitement au cisplatine. **PSO :** traitement au psoralène. **ABA:** induction de sites abasiques. **U :** introduction d'uracile.

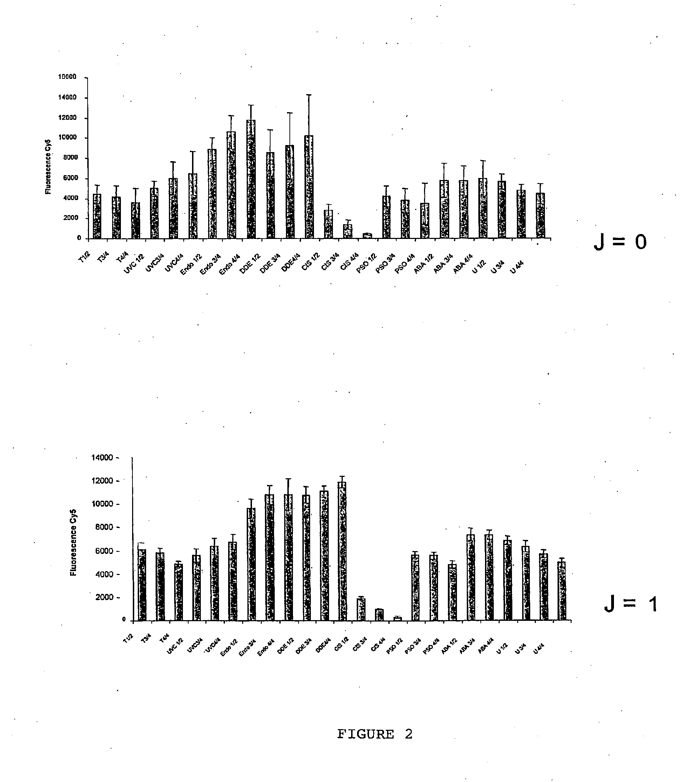

- la figure 2 illustre la stabilité à + 4°C d'un ADN superenroulé lésé, immobilisé sur des lames de verre recouvertes de poly-L-Lysine. De l'ADN plasmidique superenroulé présentant des lésions induites par différents agents physiques ou chimiques a été préparé comme décrit à l'exemple 1, puis dilué à l'aide de plasmide contrôle (non-traité) pour conserver la concentration totale de plasmide dans le tampon PBS à 40 µg/ml : uniquement du plasmide lésé **(4/4)**, 30 µg/ml de plasmide lésé et 10 µg/ml de plasmide contrôle **(3/4) ;** 20 µg/ml de plasmide lésé et 20 µg/ml de plasmide contrôle **(1/2).** De l'ADN plasmidique non-traité a été utilisé comme contrôle. Les échantillons d'ADN ont été déposés sur des lames de verre recouvertes de poly-L-lysine, puis la réparation de l'ADN a été évaluée immédiatement après le dépôt par mesure de l'émission de fluorescence (J₀) ou les lames ont été conservées à +4°C pendant 1 jour (J₁,) avant d'évaluer la réparation de l'ADN. **T :** contrôle. **UVC :** irradiation aux UVC (0,3 J/cm²). **endo :** traitement à l'endoperoxyde de naphtalène. **DDE :** traitement au trans, trans-2,4-décadiénal. **CIS :** traitement au cisplatine. **PSO :** traitement au psoralène. **ABA :** induction de sites abasiques. **U :** introduction d'uracile.

Il doit être entendu, toutefois, que ces exemples sont donnés uniquement à titre d'illustration de l'objet de l'invention, dont ils ne constituent en aucune manière une limitation.

### Exemple : Utilisation d'un ADN immobilisé dans un hydrogel comme substrat pour analyser la réparation de l'ADN

Le principe du test de réparation de l'ADN qui a été utilisé pour comparer la réparation de l'ADN immobilisé sur un support recouvert, soit de poly-L-lysine, soit d'hydrogel, est décrit dans la Demande de Brevet FR 0216435.

### 1) Matériel et méthodes

### a) Préparation d'ADN superenroulé comprenant des lésions

Le plasmide (pBluescript II, STRATAGENE) est produit par transformation de la souche XL1 blue MRF' d'*E. coli* (STRATAGENE) selon le protocole fourni par le fournisseur. Le plasmide est purifié en utilisant le kit Plasmid midi kit^{™} (QIAGEN) et dilué dans du tampon PBS (10 mM phosphate, pH 7,4, 137 mM NaCl, 2 mM KCl).

Le plasmide est traité avec différents agents physiques ou chimiques afin d'y introduire des lésions, à savoir :

### - irradiation aux UVC (UVC)

Le plasmide en solution dans du PBS (25 µl ; 40 µg/ml) est irradié avec une lampe émettant à 254 nm. La dose reçue est de 0,3 J/cm² (figures 1 et 2) ou bien de 0,03, 0,06 et 0,12 J/cm² (figure 3).

### - traitement à l'endoperoxyde de naphtalène (Endo)

20 µl d'endoperoxide de naphtalène à 50 mM (1,4-endoperoxyde de N,N',-di(2,3-dihydroxypropyl)-1,4-naphtalènedipropanamide, préparé selon le protocole décrit dans J. Biol. Chem., 2000, 275, 40601-40604) sont incubés avec 200 µl de plasmide à 1 mg/ml dans du PBS, pendant 2 heures à 37°C.

### - traitement au trans-trans-2,4-decadiénal (DDE)

200 µl de plasmide à 1 mg/ml dans du PBS sont mélangés avec 200 µl de tampon carbonate/bicarbonate 0,2 M, pH 9,2 et 200 µl de tetrahydrofurane, auxquels on ajoute 4 µl de DDE (5,7 M) et 12 µl de H₂O₂ (8,8 M). Le mélange est incubé à 50°C pendant 16 heures, à l'obscurité. Le DDE est ensuite éliminé par deux extractions au dichlorométhane.

### - traitement au cisplatine (CIS)

150 µl de plasmide à 1 mg/ml dans du PBS sont traités avec 1 µl d'une solution de cis-diamine dichloroplatine (II) à 15 mg/ml dans du diméthylesulfoxyde (DMSO), pendant 2 heures à 37°C.

### - traitement au psoralène (PSO)

150 µl de plasmide à 1 mg/ml dans du PBS sont mélangés avec 20 µl d'une solution de psoralène amine à 120 µM, et irradiés à 365 nm à une dose de 1,48 J/cm².

### - création de sites abasiques (ABA)

100 µl de plasmide à 1 mg/ml sont incubés dans du citrate de sodium à 0,05 M, pH 4,8 contenant 12 µl de KCl (2 M), pendant 4 heures à 70°C.

### - introduction d'uracile (U)

Le plasmide (pBluescript II, STRATAGENE) est produit par transformation d'une souche d'*E. coli* déficiente pour l'activité uracil-ADN-glycosylase (UNG⁻), selon le protocole fourni par le fournisseur. Le plasmide est purifié en utilisant le kit Plasmid midi kit^{™} (QIAGEN) puis sur gradient de saccharose.

Le plasmide traité est ensuite précipité, purifié sur gradient de saccharose, et les fractions contenant au moins 90 % de plasmide superenroulé sont récoltées, comme décrit dans la Demande FR 0216435. Les différentes préparations d'ADN superenroulé comprenant des lésions, ainsi que le plasmide contrôle non-traité comprenant de l'ADN superenroulé sans lésions, sont dilués à 40 µg/ml dans du PBS.

### b) Préparation des supports et dépôt de l'ADN

Les lames de verre recouvertes de poly-L-lysine sont fournies par VWR International.

### b₁) préparation des lames de verre recouvertes d'un hydrogel de polyacrylamide

Des lames de verre (76 x 26 mm) sont placées sur un portoir, puis incubées dans une solution comprenant 4 ml de Bind-Silane (3-méthacryloxypropyl-triméthoxysilane) et 220 µl d' acide acétique glacial dans un volume total d'un litre d'eau distillée, pendant 1 heure, sous agitation. Les lames sont ensuite rincées trois fois avec de l'eau distillée et une fois avec de l'éthanol, puis séchées sous une hotte aspirante.

L'hydrogel de polyacrylamide est une solution aqueuse, pouvant également être préparée à partir d'un tampon PBS (ou d'un tampon TBE), contenant : 10 % d'un mélange d'acrylamide et de méthylène bisacrylamide 19:1, 0,8 % d'agarose à bas point de fusion (low-melting agarose ; TEBU-BIO), 0,06 % de persulfate d'ammonium, 0,065 % de TEMED ((N,N,N',N'-tetraméthyléthylène diamine). 100 µl de cette solution sont déposés uniformément sur une lame de verre (76 x 26 mm), préparée comme précédemment, puis étalés de manière fine et homogène à l'aide d'une lamelle de verre. La lame recouverte de la lamelle est chauffée à 40°C pendant 5 minutes, pour permettre au gel de se figer. La lamelle de verre est alors retirée, puis la lame portant le gel est chauffée de nouveau à 40°C, pendant 3 minutes, afin de sécher l'hydrogel. Les lames recouvertes d'hydrogel de polyacrylamide sont conservées à + 4°C. De préférence, elles sont conservées au moins 24 h à + 4°C avant d'être utilisées.

### b₂) Dépôt des échantillons d'ADN

La concentration totale en plasmide dans les échantillons est de 40 µg/ml dans le tampon PBS et plus précisément en référence aux figures 1 et 2 : uniquement du plasmide lésé (4/4) ; 30 µg/ml de plasmide lésé et 10 µg/ml de plasmide contrôle (3/4) ; 20 µg/ml de plasmide lésé et 20 µg/ml de plasmide contrôle (1/2). L'ADN plasmidique superenroulé sans lésion est utilisé comme contrôle.

Trois gouttes d'échantillon d'ADN (volume total d'environ 500 picolitres) sont déposées sur chaque zone de la lame, à l'aide d'un robot piézo-électrique (Scieflexarrayer, SCIENION).

Les lames recouvertes de poly-L-Lysine sont utilisées immédiatement après le dépôt (J₀) ou conservées 24 h à 4°C (J₁) avant d'analyser la réparation de l'ADN (J₁).

Après immobilisation passive de l'ADN, à 4°C, pendant 24 h, les lames recouvertes d'hydrogel sont utilisées pour analyser la réparation de l'ADN (J₁), ou conservées à la même température pendant une durée variable, avant d'analyser la réparation de l'ADN (J₃, J₇₁, J₈₅).

### c) Réaction de réparation et mesure du signal de réparation

Une solution (30 µl) contenant un lysat cellulaire susceptible de contenir des systèmes de réparation de l'ADN (25 µl ; 0,6 mg/ml de protéines d'extraits nucléaires de cellules HeLa; CIL BIOTECH) et différents composés nécessaires à l'accomplissement de la réaction de réparation par les protéines du milieu biologique (5 µl de tampon ATG (200 mM Hepes KOH pH 7,8, 356 mM MgCl₂, 2,5 mM DTT, 1,25 pM dATP, 1,25 pM dGTP, 1,25 pM TTP, 17 % glycerol, 10 mM EDTA, 250 µg/ml créatine phosphokinase, 50 mM phosphocréatine, 10 mM ATP, 1,25 µM dCTP-Cy5)), est déposée sur chaque zone de la lame contenant les différents échantillons d'ADN immobilisé. Les lames sont incubées pendant 3 heures à 30°C. Les lames sont ensuite lavées successivement dans du PBS contenant 0,5 % de Tween 20 (2 lavages de 3 min) et dans de l'eau distillée (2 lavages de 3 min). Après séchage par centrifugation (800 rpm, 3 min), le signal fluorescent est mesuré (Scanner Genepix 4200A, AXON) et analysé à l'aide du logiciel Genepix Pro 5.1 (AXON). Les signaux sont normalisés par rapport à la médiane des signaux de chaque zone.

### 2) Résultats

### a) amélioration de la sensibilité de l'évaluation de la réparation de l'ADN

L'ADN immobilisé sur une lame recouverte d'hydrogel permet d'augmenter la sensibilité de détection de l'activité de réparation de l'ADN, comme le montre la comparaison des mesures de réparation de l'ADN effectuées après immobilisation passive des échantillons d'ADN (Figure 1).

En effet, par comparaison avec l'utilisation de poly-L-lysine (signaux compris entre 9000 et 12000 unités arbitraires pour les différentes lésions à J₀), l'utilisation de l'hydrogel de polyacrylamide permet d'obtenir des signaux de réparation de l'ADN compris dans une plus grande gamme de valeurs (signaux compris entre 11000 et 20000 unités arbitraires pour les différentes lésions à J₁), et ce quel que soit le type de lésion de l'ADN dont la réparation est étudiée. Cette différence pourrait s'expliquer par une amélioration de la quantité et/ou de la qualité de l'ADN immobilisé dans l'hydrogel et de l'efficacité de la réaction de réparation dans ce milieu.

### b) stabilité de l'ADN immobilisé

L'ADN immobilisé sur une lame recouverte d'hydrogel est stable pendant plus de 2 mois, comme le montre les résultats des tests de réparation de l'ADN effectués à différents temps après le dépôt de l'ADN sur l'hydrogel (Figure 1). A J₇₁, l'activité de réparation de l'ADN est comparable à celle détectée à J₁, et ce quel que soit le type de lésion dont la réparation est étudiée. En outre, le bruit de fond plus faible observé à J₁ avec l'ADN contrôle reflète une meilleure préservation de la structure de l'ADN lors de l'immobilisation de l'ADN dans l'hydrogel.

Par comparaison, avec l'ADN immobilisé sur une lame recouverte de poly-L-lysine, on observe un bruit de fond plus élévé à J₀ avec l'ADN contrôle, ainsi qu'une augmentation rapide du signal de réparation de l'ADN (J₁), à la fois dans les échantillons d'ADN contrôle et d'ADN lésé indiquant l'apparition de cassures supplémentaires dans l'ADN.

## Revendications

1. Procédé d'immobilisation d'un ADN superenroulé, **caractérisé en ce qu'**il comprend les étapes suivantes :
- le dépôt d'un échantillon d'ADN superenroulé à la surface d'un film de polymère poreux constitué d'une couche mince d'une matrice tridimensionnelle comprenant des chaînes d'un ou plusieurs polymères et un solvant, laquelle matrice incluant des interstices pouvant contenir des macromolécules comme l'ADN, et
- l'immobilisation de l'ADN superenroulé par diffusion passive dans ledit film de polymère poreux.

2. Procédé selon la revendication 1, **caractérisé en ce que** le polymère poreux est un hydrogel de polyacrylamide.

3. Procédé selon la revendication 2, **caractérisé en ce que** l'hydrogel de polyacrylamide comprend 5 à 15 % d'un mélange acrylamide : méthylène bisacrylamide 50:1 à 5:1.

4. Procédé selon la revendication 3, **caractérisé en ce que** l'hydrogel de polyacrylamide comprend 5 à 15 % d'un mélange acrylamide : méthylène bisacrylamide 19:1.

5. Procédé selon l'une quelconque des revendications 1 à 4 **caractérisé en ce que** le film de polymère comprend 0,1 % à 5 % d'adsorbant(s) de l'ADN.

6. Procédé selon la revendication 5, **caractérisé en ce que** l'adsorbant est un polysaccharide.

7. Procédé selon la revendication 6, **caractérisé en ce que** l'adsorbant est de l'agarose.

8. Procédé selon la revendication 7, **caractérisé en ce que** l'adsorbant est de l'agarose à bas point de fusion.

9. Procédé selon l'une quelconque des revendications 4 à 8, **caractérisé en ce que** l'hydrogel de polyacrylamide comprend 10% d'un mélange d'acrylamide : méthylène bisacrylamide 19:1 et 0,8 % d'agarose à bas point de fusion.

10. Procédé selon l'une quelconque des revendications 1 à 9, **caractérisé en ce que** ledit ADN superenroulé est un plasmide.

11. Procédé selon l'une quelconque des revendications 1 à 10, **caractérisé en ce que** ledit ADN superenroulé comprend des lésions induites par un agent génotoxique.

12. Procédé selon l'une quelconque des revendications 1 à 11, **caractérisé en ce que** le film de polymère poreux est déposé sur un support approprié.

13. Procédé selon la revendication 12, **caractérisé en ce que** ledit support est une lame de verre.

14. Support recouvert d'un film de polymère poreux comprenant un ADN superenroulé immobilisé, susceptible d'être obtenu par le procédé selon la revendication 12 ou la revendication 13.

15. Support selon la revendication 14, **caractérisé en ce qu'**il s'agit d'une lame de verre recouverte d'un hydrogel de polyacrylamide comprenant un ADN superenroulé lésé.

16. Utilisation d'une composition aqueuse comprenant 10 % d'un mélange d'acrylamide: méthylène bisacrylamide 19:1 et 0,8 % d'agarose à bas point de fusion pour immobiliser de l'ADN superenroulé.

17. Utilisation d'un ADN superenroulé immobilisé dans un film de polymère poreux tel que défini à l'une quelconque des revendications 1 à 13, comme substrat pour analyser la réparation de l'ADN.

## Claims

1. A method for immobilizing a supercoiled DNA, **characterized in that** it comprises the following steps:
- depositing a supercoiled DNA sample on the surface of a porous polymer film constituted by a thin layer of a three-dimensional matrix comprising chains of one or more polymers and a solvent, which matrix includes interstices that can contain macromolecules such as DNA, and
- immobilizing the supercoiled DNA by passive diffusion in said porous polymer film.

2. The method as claimed in claim 1, **characterized in that** the porous polymer is a polyacrylamide hydrogel.

3. The method as claimed in claim 2, **characterized in that** the polyacrylamide hydrogel comprises 5% to 15% of a 50:1 to 5:1 acrylamide:methylenebisacrylamide mixture.

4. The method as claimed in claim 3, **characterized in that** the polyacrylamide hydrogel comprises 5% to 15% of a 19:1 acrylamide:methylenebisacrylamide mixture.

5. The method as claimed in any one of claims 1 to 4, **characterized in that** the polymer film comprises 0.1% to 5% of DNA adsorbent(s).

6. The method as claimed in claim 5, **characterized in that** the adsorbent is a polysaccharide.

7. The method as claimed in claim 6, **characterized in that** the adsorbent is agarose.

8. The method as claimed in claim 7, **characterized in that** the adsorbent is low-melting-point agarose.

9. The method as claimed in any one of claims 4 to 8, **characterized in that** the polyacrylamide hydrogel comprises 10% of a 19:1 acrylamide:methylenebisacrylamide mixture and 0.8% of low-melting-point agarose.

10. The method as claimed in any one of claims 1 to 9, **characterized in that** said supercoiled DNA is a plasmid.

11. The method as claimed in any one of claims 1 to 10, **characterized in that** said supercoiled DNA contains damage induced by a genotoxic agent.

12. The method as claimed in any one of claims 1 to 11, **characterized in that** the porous polymer film is deposited onto an appropriate support.

13. The method as claimed in claim 12, **characterized in that** said support is a glass slide.

14. A support coated with a porous polymer film, comprising a immobilized supercoiled DNA, which may be obtained by means of the method as claimed in claim 12 or claim 13.

15. The support as claimed in claim 14, **characterized in that** it is a glass slide coated with a polyacrylamide hydrogel comprising a damaged supercoiled DNA.

16. The use of an aqueous composition comprising 10% of a 19:1 acrylamide:methylenebisacrylamide mixture and 0.8% of low-melting-point agarose, for immobilizing supercoiled DNA.

17. The use of a supercoiled DNA immobilized in a porous polymer film as defined in any one of claims 1 to 13, as substrate for analyzing DNA repair.

## Patentansprüche

1. Verfahren zur Immobilisierung einer superhelikalen DNA, **dadurch gekennzeichnet, dass** es die folgenden Stufen umfasst:
- Abscheidung einer Probe von superhelikaler DNA auf der Oberfläche eines porösen Polymerfilms, der aus einer dünnen Schicht einer dreidimensionalen Matrix besteht, die Ketten eines oder mehrerer Polymere und ein Lösungsmittel umfasst, wobei die Matrix Zwischenräume enthält, die Makromoleküle wie DNA enthalten können, und
- Immobilisierung der superhelikalen DNA durch passive Diffusion in den porösen Polymerfilm.

2. Verfahren nach Anspruch 1, **dadurch gekennzeichnet, dass** das poröse Polymer ein Polyacrylamid-Hydrogel ist.

3. Verfahren nach Anspruch 2, **dadurch gekennzeichnet, dass** das Polyacrylamid-Hydrogel 5 bis 15% eines Gemisches Acrylamid:Methylenbisacrylamid 50:1 bis 5:1 umfasst.

4. Verfahren nach Anspruch 3, **dadurch gekennzeichnet, dass** das Polyacrylamid-Hydrogel 5 bis 15% eines Gemisches Acrylamid:Methylenbisacrylamid 19:1 umfasst.

5. Verfahren nach einem der Ansprüche 1 bis 4, **dadurch gekennzeichnet, dass** der Polymerfilm 0,1% bis 5% Adsorbens (Adsorbenzien) für DNA umfasst.

6. Verfahren nach Anspruch 5, **dadurch gekennzeichnet, dass** das Adsorbens ein Polysaccharid ist.

7. Verfahren nach Anspruch 6, **dadurch gekennzeichnet, dass** das Adsorbens Agarose ist.

8. Verfahren nach Anspruch 7, **dadurch gekennzeichnet, dass** das Adsorbens Agarose mit niedrigem Schmelzpunkt ist.

9. Verfahren nach einem der Ansprüche 4 bis 8, **dadurch gekennzeichnet, dass** das Polyacrylamid-Hydrogel 10% eines Gemisches Acrylamid:Methylenbisacrylamid 19:1 und 0,8% Agarose mit niedrigem Schmelzpunkt umfasst.

10. Verfahren nach einem der Ansprüche 1 bis 9, **dadurch gekennzeichnet, dass** die superhelikale DNA ein Plasmid ist.

11. Verfahren nach einem der Ansprüche 1 bis 10, **dadurch gekennzeichnet, dass** die superhelikale DNA Läsionen umfasst, die durch ein genotoxisches Mittel induziert wurden.

12. Verfahren nach einem der Ansprüche 1 bis 11, **dadurch gekennzeichnet, dass** der poröse Polymerfilm auf einem geeigneten Träger abgeschieden ist.

13. Verfahren gemäß Anspruch 12, **dadurch gekennzeichnet, dass** der Träger ein Glasplättchen ist.

14. Träger, überzogen mit einem porösen Polymerfilm, umfassend eine immobilisierte superhelikale DNA, erhältlich durch das Verfahren nach Anspruch 12 oder Anspruch 13.

15. Träger nach Anspruch 14, **dadurch gekennzeichnet, dass** es sich um ein Glasplättchen handelt, überzogen mit einem Polyacrylamid-Hydrogel, das eine superhelikale beschädigte DNA umfasst.

16. Verwendung einer wässrigen Zusammensetzung, umfassend 10% eines Gemisches Acrylamid:Methylenbisacrylamid 19:1 und 0,8% Agarose mit niedrigem Schmelzpunkt, um superhelikale DNA zu immobilisieren.

17. Verwendung einer superhelikalen DNA, die in einem porösen Polymerfilm, wie er in einem der Ansprüche 1 bis 13 definiert ist, immobilisiert ist, als Substrat, um die Reparatur der DNA zu analysieren.
